# EUROPEAN PATENT APPLICATION

(11) **EP 4 564 368 A1**
(43) Date of publication of application: **04.06.2025**
(21) Application number: 23217095.1
(22) Date of filing: 15.12.2023
(51) Int. Cl.: G16H 40/63, G06F 3/0482, G06F 9/451

(54) **GRADUAL FEATURE DISCLOSURE TO FAMILIARIZE USER WITH SYSTEM**

(30) Priority: 28.11.2023 US 202363603183 P
(71) Applicant: Koninklijke Philips N.V., 5656 AG Eindhoven (NL)
(72) Inventor: HUIJBREGTS, Laurentia Johanna, Eindhoven (NL); VAN LIESHOUT, Ron Martinus Laurentius, Eindhoven (NL); MAESSEN, Ralph Theodorus Hubertus, 5656AG Eindhoven (NL); BULUT, Murtaza, Eindhoven (NL); BOUTS, Mark Jacobus Rosalie Joseph, Eindhoven (NL)
(74) Representative: Philips Intellectual Property & Standards

(57) **Abstract**

A non-transitory computer readable medium (**16**) stores instructions readable and executable by an electronic processor (**14**) to perform a user interface (UI) updating method (**100**) for a medical device (**10**). The UI updating method includes receiving an update (**30**) for a UI (**28**), wherein features of the update are grouped into a plurality of chunks; assigning chunks of the plurality of chunks an active or hidden state; and presenting the UI including the features of the chunks assigned.

## Description

### FIELD OF THE INVENTION

The following relates generally to the device and systems monitoring arts, medical device education arts, medical device updating arts, and related arts.

### BACKGROUND OF THE INVENTION

A computerized medical device such as a patient monitor, a medical imaging device, a mechanical ventilation system, or so forth typically includes a user interface (e.g., a graphical user interface, or GUI) that is implemented using a display of the medical device and one or more user input devices such as a mouse, trackball, keyboard, touch-sensitive surface of the display, various combinations thereof, or so forth. The user interface facilitates complex interactions between the user and the medical device. For example, a medical device that measures patient vital signs or other patient data can process the patient data and present one or more derived values that can be of more readily apparent clinical significance than the raw measured patient data. The data can be presented in various ways, such as by numerical values, graphs, pie charts, or so forth. The user interface can provide various user dialogs to facilitate user configuration of the medical device, and the computerization of the medical device also enables varied ways of configuring the medical device. Furthermore, because the user interface is software driven, it can be improved over time by pushing updates to the medical device that provide new features that modify the user interface of the medical device. This can also occur in conjunction with added hardware - for example, a patient monitor may be improved by adding a new type of sensor, and the user Interface is also updated to maximize the usability of the new sensor and data acquired by the sensor.

Often, however, users are reluctant to take advantage of new features in a new or upgraded electronic medical system. This can be due to factors such as unfamiliarity with the new features, and concern about the accuracy of patient data or analyses presented via the new features. Reluctance can be increased in certain settings, such as an intensive care unit (ICU), surgical suite, or other critical care setting where users (e.g., doctors, nurses, et cetera) rely on following a familiar routine and employing medical devices with static user interfaces to ensure mistakes are not made. This can lead to user dissatisfaction with the upgrade, in spite of the useful new capabilities the upgrade may provide.

The following discloses certain improvements to overcome these problems and others.

### SUMMARY OF THE INVENTION

In some embodiments disclosed herein, a medical device includes a display; an input interface via which patient data is received; and an electronic processor implementing a user interface (UI) in which a representation of the patient data is presented on the display. The electronic processor is further programmed to perform an update of the medical device including receiving an update for the medical device, the update comprising executable code for adding a plurality of features that modify the UI, the features being grouped into chunks; generating a data structure in which the plurality of chunks are assigned an active or hidden state; progressively implementing the update of the medical device by presenting the UI modified by the features of the chunks assigned the active state and not modified by the features of the chunks assigned the hidden state; and updating the data structure in response to an update trigger to reassign a triggered chunk of the plurality of chunks from the hidden state to the active state, whereby after the updating of the medical device the UI is presented modified by the features of the triggered chunk.

In some embodiments disclosed herein, a non-transitory computer readable medium stores instructions readable and executable by an electronic processor to perform a UI updating method for a medical device. The UI updating method includes receiving an update for a UI, wherein features of the update are grouped into a plurality of chunks; assigning chunks of the plurality of chunks an active or hidden state; and presenting the UI including the features of the chunks assigned.

In some embodiments disclosed herein, a medical device updating method includes grouping features of a received update of the medical device into a plurality of chunks; assigning chunks of the plurality of chunks an active or hidden state; presenting a UI of the medical device modified by the features of the chunks assigned the active state and not modified by the features of the chunks assigned the hidden state; analyzing usage of the presented UI by a user; selecting a chunk assigned the hidden state based on the analysis of the usage of the presented UI by the user; reassigning the selected chunk from the hidden state to the active state; and after the reassigning, continuing the presenting of the UI modified by the features of the chunks assigned the active state and not modified by the features of the chunks assigned the hidden state, whereby modification of the UI produced by one or more features grouped into the selected chunk are revealed.

One advantage resides in gradually rolling out and/or activating new features for an updated medical device.

Another advantage resides in determining a usage of features of an updated medical device before introducing new features to the medical device.

Another advantage resides in reducing user confusion and fatigue for new features of an updated medical device.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically illustrates a medical monitor in accordance with the present disclosure.
Fig. 2 diagrammatically illustrates a user interface updating method using the medical monitor of Fig. 1.
Figs. 3 and 4 show additional method embodiments for gradually updating the user interface of the medical monitor of Fig. 1.

### DETAILED DESCRIPTION OF EMBODIMENTS

The following discloses a system implementing an automated gradual rollout of new features, in a manner that facilitates user adoption. For example, individual features may be presented at predefined intervals, so that the user is not overwhelmed with many changes to the user interface. In another approach, usage of a newly added feature *fᵢ* is monitored, and a next feature *f*_{*i*+1} is added after the monitored usage indicates users have accepted feature *fᵢ*. Again, by rolling out features gradually in this way, users are not overwhelmed by an abrupt change in the user interface.

To implement the approach, the new features are divided into chunks. Each chunk includes one or more new features. Each chunk can be added as a unit, although there may be chunk dependencies. For example, a chunk *C_{y}* may rely upon a chunk *Cₓ* so that the chunk *C_{y}* can only be added after the chunk *Cₓ* is added (or chunks *Cₓ* and *C_{y}* could be added together). The software implementing the user interface is modified to include a data structure storing the state of each chunk (active or hidden), an optional chunk dependencies data structure (this could be omitted if there are no chunk dependencies), and a feature rollout module that controls the gradual rollout of features by controlling when various chunks are switched from hidden to active. In one approach, the rollout module activates chunks in accord with a time sequence (e.g., activate chunk *C*₁ at time *t*₁; activate chunk *C*₂ at time *t*₂ > *t*₁; activate chunk *C*₃ at time *t*₃ > *t*₂; ....). In another example, the time sequence can include a granularity related to an employee shift and/or a workflow.

In other approaches, the feature rollout module monitors usage of features of the user interface to determine when to activate new chunks. For example, the next chunk could be activated when monitoring of user interaction with the user interface indicates users have accepted the features provided by previously activated chunks.

In other approaches, the activation of chunks may not follow a predetermined sequence. Rather, the feature rollout module could monitor usage of the user interface to determine both when to activate new chunks and which new chunk to activate next. Since the goal is generally to achieve a gradual rollout, if the two or more chunks could be activated next, the feature rollout modules can decide which chunk to actually activate based on factors such as perceived usefulness, perceived stress introduced by introducing the various chunks, effect of each new chunk on the visual appearance (e.g. clutter) of the display, or so forth.

In the foregoing examples, the gradual rollout of new features can be different for different user entities, where a user entity could be an individual user or a group of users such as a medical department, a defined clinical team, or so forth, or could be further based on training records, including for example consumption of micro-learnings on the specific feature. As an example of the latter, a feature could be rolled out to individuals once they complete a relevant training session. Consequently, the gradual rollout could be occurring concurrently and/or sequentially in different medical departments, or with different clinical teams, different trainee groups, or so forth. Information determined by monitoring adoption of new chunks by one user entity could therefore serve as input for determining activation of chunks during the gradual rollout for another user entity. As just one example, if in previous gradual rollouts adoption of a particular chunk experienced strong resistance by users, then that chunk could be activated later in subsequent gradual rollouts. Additionally, individuals should be looked at based on the different systems that they interact with and the feature updates on going and/or planned for the group of systems for that individual. This can help with both rate of feature roll-out as well as aligning any similar features across the systems to aid in the learning and familiarity of the new features.

A further consideration is that a given user entity should experience a smooth rollout. For example, if the user entities are grouped such that a given clinician is a member of two user entities, then it may be undesirable for that clinician to experience the benefit of an activated feature when working as part of one user entity but to then have that feature hidden when working as part of another user entity. This could be addressed by designing the user entities to ensure each clinician is a member of a single user entity, or by adjusting the activated features for the specific clinician (if, for example, the clinician logs in then the same activated features can be activated for that clinician regardless of which user entity the clinician is currently working under).

In other variants, chunks could be activated immediately (i.e., prematurely when compared with the programmatic rollout) based on clinical need. This can be based on data analysis, looking at the type of clinical cases performed, scheduled, or both. Additionally or alternatively, chunks could be activated based on clinical need as indicated by a feature list usage rate to determine who needs features rolled-out and in what order to best increase the overall level of care provided. For example, in the illustrative case of a patient monitor in which the chunks provide hemodynamic monitoring display features, if analysis of patient vital signs indicates the patient is undergoing acute hemodynamic instability, then features that are useful for diagnosing/treating that instability might be activated immediately in that case. In a similar manner, if the user is provided with instructional materials on the medical system, all features (including the hidden features) can be described in those materials, and a user interface settings dialog could be provided to enable users to manually activate currently hidden features as desired. In another example, a predictive algorithm can be implemented, so that hemodynamic instability (or another patient condition) is predicted before it actually happens. Then the system can already introduce the feature(s) and the user will still have some time to get used to the feature(s) before they are actually needed.

In yet further variants, features could be deactivated (i.e., switched back from activated to hidden) if monitored usage indicates the feature is not being adopted by users, or if monitored outcomes indicate the feature is not benefiting patients.

In still yet further variants, if the medical system incorporates user biofeedback such as gaze tracking, this information can be used in assessing adoption of the features of newly activated chunks, e.g. by detecting how frequently the user looks at a newly provided visual representation of patient data, or a frequency of how the user looks at the newly provided visual representation of patient data, or a dwelling time of how the user looks at the newly provided visual representation of patient data. As another example, if the user has the ability to hide a feature by selecting a "Hide" button or the like, then statistics on users hiding (or not hiding) the feature can be tracked and used in assessing user adoption of the feature. Additionally, a rate of consumption can be determined for each clinician (or each group of clinicians) on the basis of, for example, individualized gaze tracking or another monitoring mechanism (e.g., a timer timing how long it takes for the users to consume the content). The individual user (or user group) adoption of the new features is compared over time. For those users (or groups) with high levels of adoption, the rate at which new chunks/features are rolled out can be greater. In general, the rate of rollout of new chunks/features can be balanced so as not to frustrate the users with constant change, but also not too much at one time. Such balancing can be done on a per-clinician basis, or on a per clinician group basis, or using a combination of individual/group analyses.

The gradual rollout can be employed in the context of a newly deployed electronic medical device for which all features are initially included. However, the disclosed gradual rollout approaches could also be employed to gradually activate features of a major upgrade (e.g., pushed to the medical system via the Internet) with enough new user interface features that there is a potential to overwhelm users.

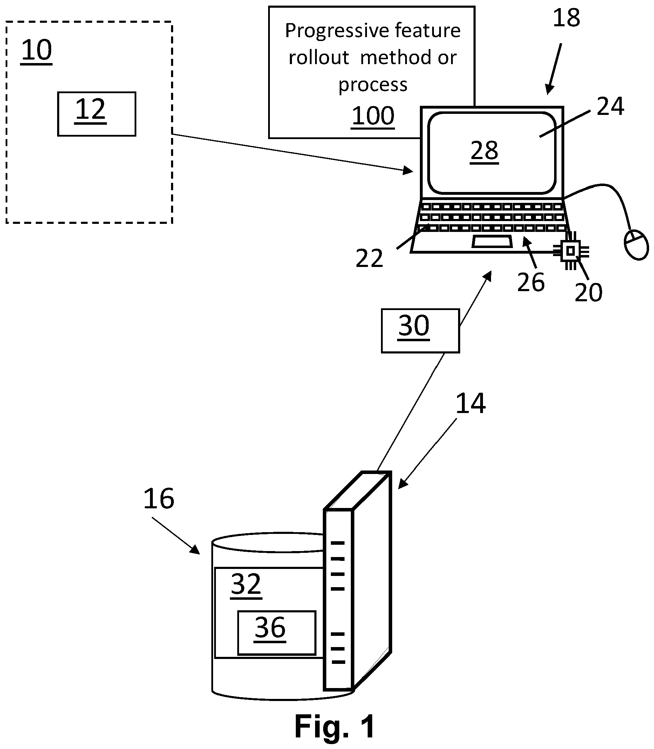

With reference to Fig. 1, an illustrative medical device **10** is shown. The medical device **10** includes, or is operatively connected to, an electronic processing device **18,** such as a workstation computer, or more generally a computer, a mobile device (e.g., a tablet computer), a built-in user interface **18** that is built into the medical device **10** (e.g., a patient monitor having a built-in display and built-in keypad, buttons, touch-sensitive display overlay, various combinations thereof, and/or the like). The electronic processing device **18** includes typical components for enabling user interfacing, such as an electronic processor **20** (e.g., a microprocessor), at least one user input device (e.g., a mouse, a keyboard, a trackball, and/or the like) **22,** and a display device **24** (e.g., an LCD display, plasma display, cathode ray tube display, and/or so forth). In some embodiments, the display device **24** can be a separate component from the electronic processing device **18** or may include two or more display devices. To display color-coded image(s) and/or data, the display device **24** should typically be a color display device, although a monochrome display is also contemplated.

A non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the electronic processing device **18,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a visualization of a user interface (UI) **28,** which is typically (although not necessarily) a graphical user interface (GUI) **28,** for display on the display device **24.**

The electronic processing device **18** can comprise a built-in or separate controller of the medical device **10.** The medical device **10** includes an input interface **12** via which patient data is received. The medical device **10** (shown schematically in Fig. 1 as a rectangle) can comprise a patient device, for example, a medical imaging device (e.g., magnetic resonance (MR), computed tomography (CT), image guided therapy (IGT) systems, ultrasound, X-ray, positron emission tomography (PET), single photon emission computed tomography (SPECT), ultrasound (US), and so forth) and the input interface **12** includes imaging data acquisition hardware configured to receive the patient data as patient medical images. In another example, the medical device **10** can comprise a patient monitor with a vital sign sensor interface **12** configured to receive patient vital sign data. In another example, the medical device **10** can comprise a mechanical ventilator with a respiratory sensor interface **12** configured to receive patient respiratory data. In another example, the medical device **10** can comprise a medical records workstation with a hospital information technology (IT) network **12** configured to receive patient medical record data. In another example, the medical device **10** can comprise a patient doctor management system (PDMS) with an electronic medical record (EMR) interface **12** configured to receive patient medical data from the EMR. These are merely illustrative examples and should not be construed as limiting.

As shown in Fig. 1, the medical device **10** includes, or is accessible by, a server computer **14.** The server computer **14** comprises a computer or other programmable electronic device that includes or has operative access to a non-transitory computer readable medium. The server computer **14** communicates with the medical device **10** via the Internet, optionally along with one or more local area networks (LAN) or wide-area networks (WAN) It will be appreciated that the server computer **14** could be implemented as a plurality of server computers, e.g., interconnected to form a server cluster, cloud computing resource, or so forth, to perform more complex computational tasks. The server computer **14** includes a database **16** comprising a magnetic disk, RAID, or other magnetic storage medium; a solid-state drive, flash drive, electronically erasable read-only memory (EEROM) or other electronic memory; an optical disk or other optical storage; various combinations thereof; or so forth.

The database **16** stores instructions executable by the server computer **14** (and/or by the electronic processing device **18)** to push an update **30** to the medical device **10.** The database **16** may, for example, be maintained by a vendor of the medical device **10,** who pushes an update **30** of the medical device **10,** for example via the Internet and/or a hospital IT network. In another contemplated embodiment (not shown), the update **30** could be provided on a physical medium such as a USB drive, optical disk, or the like which is loaded into a suitable interface (e.g., USB port, optical disk reader, et cetera) of the medical device **10** to apply the update **30.** The update **30** of the medical device modifies the UI **28,** either directly or indirectly. As an example of an update of the medical device **10** that directly modifies the UI **28,** the update may modify a graph of patient data presented on the UI **28** to more clearly show clinically important features. As an example of an update of the medical device **10** that indirectly modifies the UI **28,** the update **30** may add calculation of a clinical metric not previously calculated by the medical device **10,** and the UI **28** is modified by displaying this new clinical metric. In some cases, the modification of the UI **28** by the update **30** of the medical device **10** could entail simplification of the UI **28,** for example by removing a graph or other patient data presentation that has been determined to be confusing or otherwise unhelpful to clinicians.

The received update **30** is applied to update the medical device **10.** The update **30** adds features to the UI **28** or otherwise modifies the UI **28.** These features could, by way of nonlimiting illustrative example, include improved ways of displaying patient data, or improved user dialogs that simplify user operations in configuring the medical device **10.** The features could additionally or alternatively include data analysis components that analyze the medical data collected by the medical device **10** to generate and present (via the UI **28)** derived data that may have clinical significance (for example, processing heart rate data or respiratory data to detect signatures that may be indicative of certain types of cardiac or respiratory conditions). Besides updating the UI **28,** The update **30** may also update other aspects of the medical device **10,** such as providing new or updated firmware to enable or improve connection of the medical device **10** with a new type of medical sensor or other auxiliary hardware device, and this can cause modification of the **UI 28** by way of that new sensor data being included in a patient data graph, for example.

However, as disclosed herein, the update process performed at the medical device **10** (e.g., specifically at its controller **18** if the controller is separate from the rest of the medical device **10)** for applying the update **30** to update the UI **28** (and more generally to update the medical device **10)** includes performing a progressive feature rollout method or process **100** that progressively exposes (i.e. presents) features of the update in a gradual manner, so that users are not overwhelmed by changes to the UI **28** introduced by the update **30.**

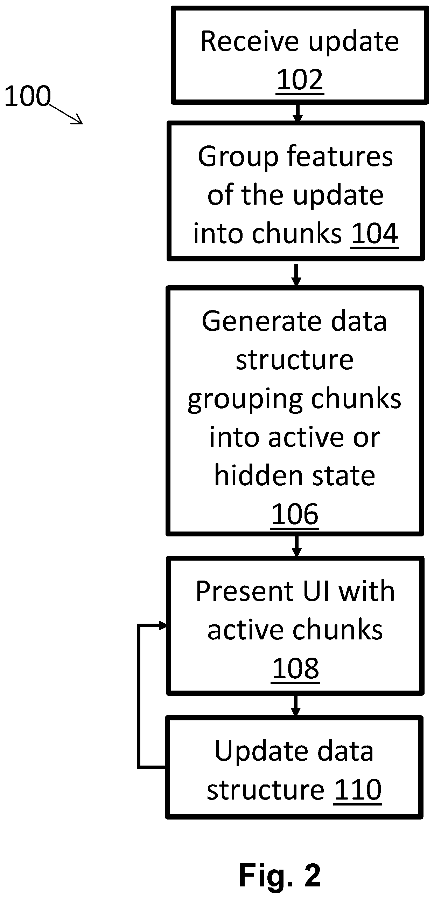

With reference to Fig. 2, and with continuing reference to Fig. 1, an illustrative embodiment of an instance of the user interface (UI) updating method **100** is diagrammatically shown as a flowchart. At an operation **102,** the medical device **10** receives an update **30** for the UI **28** (or, more generally, an update **30** for the medical device **10).** The update **30** can comprise executable code for adding a plurality of features to the UI **28.** At an optional operation **104,** features of the update **30** are grouped into chunks. (This operation **104** is optional because the update **30** as-received could already have its features grouped into chunks). The features of the plurality of chunks include one or more features in which the UI **28** presents additional (e.g. derived) information, one or more features in which the UI **28** changes a presentation format of information, one or more machine-learning processes, and/or one or more features in which the UI **28** provides user input for modifying operation of the UI **28,** and/or so forth. In one nonlimiting illustrative example, each chunk may comprise one or more programming units such as functions, software modules, objects (in an object-oriented programming, or OOP, programming framework), or so forth, in which execution of the programming unit(s) of the chunk implements the features that are grouped into that chunk. In another approach, there may not be a direct correspondence between the chunks and programming units, but rather the executable code of the update **30** may include conditional statements that implement features only if the chunks into which the features are grouped are active.

In general, each chunk is assigned a passive state or an active state. At an operation **106,** in one suitable implementation of such state assignment a data structure **32** is generated in which chunks of the plurality of chunks are assigned an active or hidden state. The data structure **32** could, by way of nonlimiting illustrative example, be a one-dimensional array with each array element corresponding to a chunk and storing a value indicative of the state of that chunk (i.e., one value indicating passive state or another value indicating active state). As another nonlimiting illustrative example, the data structure **32** could be a binary data structure such as a byte or a plurality of bytes with each bit of the binary data structure corresponding to a chunk and having either value 0=passive state or 1=active state (or vice versa). As yet another nonlimiting illustrative example, each chunk could internally store a flag (integer field, binary bit, or so forth) indicating whether the chunk is passive or active, and in this case the data structure **32** constitutes the aggregation of the flags internally stored in the various chunks of the update **30.** These are merely nonlimiting illustrative examples.

At an operation **108,** the UI **28** is presented including the features of the chunks assigned the active state and not including the features of the chunks assigned the hidden state. If the chunks correspond to logical programming units, then this can be done by activating only those logical programming units/chunks assigned the active state. If the chunks are implemented by conditional statements in the executable code of the update **30,** then the conditional statement only runs the code that implements a given feature if the chunk into which that feature is grouped is assigned the active state.

At an operation **110,** the data structure **32** is updated in response to an update trigger to reassign a triggered chunk of the plurality of chunks from the hidden state to the active state. After the updating, flow passes back to the operation **108** to continue presenting the UI **28,** now further including the features of the newly triggered chunk. The loop **108, 110** iterates, with each execution of the operation **110** reassigning a further chunk from the hidden state to the active state thereby progressively exposing (i.e., presenting, or rolling out) features of the update **30.** In some embodiments, the update trigger comprises a predetermined time interval, but as also described herein other types of triggers can be employed, such as based on analysis of user interactions with the presented UI **28.**

In some embodiments, usage of the UI **28** presented in the operation **108** is analyzed to generate the update trigger. For example, generating the update trigger comprises a predetermined dependency on the triggered chunk of another chunk that is reassigned from the hidden state to the active state. Based on the analysis of the usage of the UI **28** by the user, a criticality of a chunk is determined from the usage of the UI **28** by the user. The update trigger comprises the determined criticality satisfying a criticality trigger criterion. In a particular example, the update trigger (and determining of which chunk corresponding to the update trigger) is caused by the patient that is being taken care of (i.e. the medical urgency of the given patient, such as the type of patient or patient profile, the patient status or situation (such as being hemodynamically instable), available patient information (such as an ultrasound or lab measurement been done), and/or (planned) procedure). In another example, the update trigger (and determining of which chunk corresponding to the update trigger) can be delayed because of an alarm that needs urgent attention (i.e., which is the most important reason for delay).

In some examples (not shown in Fig. 2), the features of a chunk can be removed by reassigning the chunk from the active state to the hidden state in response to the analysis indicating usage of the chunk underruns a predetermined usage threshold. In some embodiments, the analysis of usage of the UI **28** by the user is performed by tracking a gaze of the user interacting with the UI **28.** As such, the updated UI **28** comprises an improved user interface. In some examples, a rate of consumption of the active chunks can be determined (e.g., with a tracked eye gaze of the user, with a timer to time how long it takes for the user to consume or interact with the chunks, and so forth), in which the individual user adoption of the new features is compared over time. For those users with high levels of adoption, the rate of consumption can be greater. There needs to be a balance such as not to frustrate the users with constant change, but also not too much at one time.

In some embodiments, the data structure **32** assigns the chunks to the hidden or active states on a per user or per user group basis. In this embodiment, a user or user group of the UI **28** is identified, and the UI **28** is presented to the user(s) including the chunks assigned the active state for the identified user or user group and not including the chunks assigned the hidden state for the identified user or user group. As just one nonlimiting illustrative implementation of this, the data structure **32** could be a two-dimensional array A having array elements A(x,y), where x designates the user group, y designates the chunk, and the value of array element A(x,y) stores either a passive state value (so that the feature y is not presented to users of user group x) or an active state (so that the feature y is presented to users of user group x). In another example, the data structure **32** assigns the chunks to the hidden or active states on a per user or per user group basis further based on training records, including for example consumption of micro-learnings on the specific feature. Additionally, the data structure **32** analyzes the users based on different medical systems that they interact with and the feature updates on going and/or planned for the group of systems for that particular user. This can help with both rate of feature roll-out as well as aligning any similar features across the systems to aid in the learning and familiarity of the new features.

While the method **100** is mainly intended to provide automated gradual rollout of features of the update **30,** it may optionally also allow for user interaction to manually set given features to be active or hidden. In some embodiments, therefore, a user dialog can be provided on the UI **28** via which a user can edit the data structure **32** to manually reassign chunks to the active or hidden state. By doing so, the user manually updates the UI **28** to include the features of the chunks manually assigned the active state and to not include the features of the chunks manually assigned the hidden state. In some examples, the update **30** can also be in the form of files, for example a JavaScript Object Notation (JSON) file with requirements, profiles, information, structure, tabular information, and so forth. As used herein, the update **30** comprising executable code is considered to include executable code in the form of such a JSON file or other data that modifies the execution of the software of the medical device **10.**

The following describes the medical device **10** and the method **100** in more detail. The medical device **10** can include the **UI 28** and software that comprises the functionalities that could be shown on the UI **28,** where: the total of functionalities can be divided in chunks, representing one or more components/features with a defined added (clinical) value; one or more chunks are left out on the user interface, at least for a period of time - without compromising the basic added (clinical) value; when a chunk is added to or removed from the user interface is determined by at least one of the following: the level of familiarization of the user with the chunks that have already been shown on the user interface, the need for the chunk in the clinical situation (e.g. related to the patient type and/or status), and the added or reduced (clinical) value. The added features to the UI **28** can be functions / buttons / tabs / menus / tap & slide options / graphs, etc. or combinations thereof. They are thus UI-related.

The updated medical device **10,** when it gets introduced (that is, when the update **30** is first installed), starts simple. It may start resembling "old" software that has been used before in the particular hospital, which already has a basic (clinical) value. For example, it may resemble their currently used patient monitor, with as extra features only the feature of viewing trendlines of physiological parameters, interventions and limited patient information.

In this way, the user gets for example used to (for example) grey, yellow and red color-coding of the trendlines as part of the first rolled-out feature. These illustrative example colors respectively stand for parameter in normal range (gray), parameter in yellow alarm range, and parameter in red alarm range. After some time, when the user has familiarized himself with the meaning of the colors and the view as such, one or more interaction options can get introduced. Next in line could be a caret down and caret up user dialog buttons to enable switching to a collapsed view and back.

After the user is used to the new options, more and more features may be added (i.e. rolled out), such as a menu in a left bottom corner, which can only contain 'Trends' (and hence does not seem like a menu yet), could have one or more additions, like `Set watchlist,' `Set AES profile', . These additional options in the menu constitute further features that could be added all at once (by being grouped into a single chunk) or one-by-one (each being in a different chunk), or two or three at the same time by variously grouping the menu items/features into various chunks.

Even later, further features constituting more tabs could be added; for example, a further feature that may be rolled out in the **UI 28** can be a `Patient tab', while the user interface at a later time may have further features rolled out to show one or more extra tabs at the top of the display, like `Event', and 'Diagnose'. Each of these tabs may start simple and add extra functions / buttons / tabs / menus / tap & slide options / graphs later on.

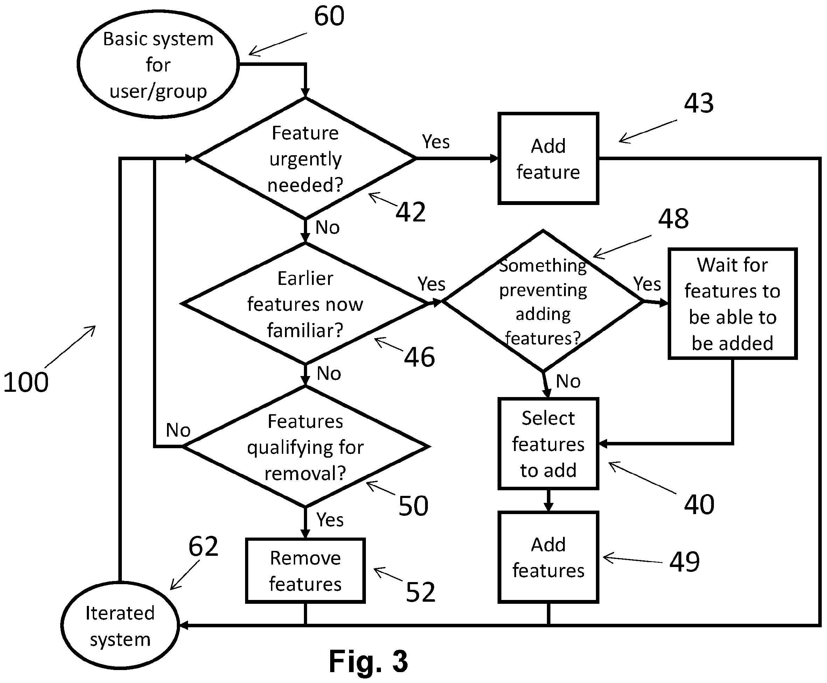

Fig. 3 shows another embodiment of the progressive feature rollout method or process **100.** As shown in Fig. 3, the medical device **10** includes a selection module **40** configured to select which features to add to the UI **28.** The selection of which feature(s) to add by the selection module **40** might follow a default order. This default order might have been programmed because it seems quite logical to first learn all possible features on one tab and then only later start to learn about other tabs.

However, in this embodiment the order might change depending on the situation. Fig. 3 shows an approach in which at a decision block **42** it is determined whether a feature is urgently needed, for example because the patient's condition is failing in a way that a new derived parameter would reveal. If so, then a flow of the method **100** passes to an operation **43** in which that feature is immediately presented, by reassigning its chunk from hidden state to active state. This can be based on data analysis, looking at the type of clinical cases performed, scheduled, or both either alone or in conjunction with a feature list usage rate to determine who needs features rolled-out and in what order to best increase the overall level of care provided. For example, in some contemplated embodiments the decision block **42** may access a hospital scheduling system to determine the tasks a particular clinician will be performing using the UI **28,** to determine which features are likely to be urgently needed by that particular clinician.

If no urgency exists, then the flow passes to a decision block **46** which determines, based on analysis of user interaction with the presented UI **28,** whether the already-presented features have become sufficiently familiar to confront the user with one or more new features. If so, then at an operation **48** it is determined whether the features can be added, or whether something prevents rolling out the feature now (for example, it may be undesirable to roll out a new feature during a critical medical emergency situation, or when a mobile medical device is being used in the intensive care unit). Assuming the check **48** is passed, then the flow passes to the operation **40** to select the feature(s) to add, and at an operation **49** the corresponding chunk is reassigned from passive state to active state so that as the UI continues to be presented the new feature(s) are rolled out. Optionally, the progressive rollout method **100** may also allow for removing features if they qualify for removal at an operation **50.** For example, if analysis of user interactions with the presented UI **28** indicate a feature is not being used and may be contributing to clutter of the UI display, then operation **50** may detect this, and the flow then passes to an operation **52** where the feature is removed by reassigning the corresponding chunk from active state to passive state, so that going forward the presented UI **28** will no longer show this feature. As shown in Fig. 3, this processing is iterative; initially, a basic UI **60** is presented with only an initial set of new features, and at each iteration of an iterated system **62** additional features may be added (or in some embodiments some iterations may remove features by the path **50, 52).** Aspects of the embodiment of the method **100** shown in Fig. 3 are described in further detail next.

The urgency module **42** is configured to determine whether there is a need to prioritize a certain feature to be implemented soon or immediately. For example, a feature not yet rolled out may implement an algorithm like I (Hemodynamic Stability index (see, e.g., Rahman et al. "Early prediction of hemodynamic interventions in the intensive care unit using machine learning" Critical Care (2021) 25:388). Although not rolled out insofar as the I is not yet displayed, the I algorithm may be run on the current patient data. If this silently monitored, I suggests that the patient is declining, then the urgency module **42** determines that an Event tab for presenting I should be added, even if not all features on a Patient tab have been rolled out. In another example, if diagnostic measurements are being done, then the urgency module **42** determines that a Diagnose tab should be added, even if not all features on the Patient tab have been disclosed. In another example, a patient arrives with a certain (current or soon to be expected) condition/disease/patient profile/(planned) procedure, for which there are one or more tailored features that have not been disclosed yet, then they should get disclosed. In another example, there may be planned educational sessions about the system. When there is an upcoming educational session in which certain features of the system are going to be explained, the system might, in the one or few days before that, disclose those features so that the users get exposed to them and can ask tailored questions in the educational session. Also, right after the educational session, new features could be added, namely those that were treated in the session. In these last examples, the urgency is not a medical urgency for a given patient, but an urgency to align the rollout of features with an educational schedule relating to those features.

In the absence of any such urgency, it is preferred in some embodiments to add features at times based on the speed of learning/familiarizing of the specific user. For that, the metric **46** is used. The following are examples of the metric **46** to determine the time at which a new feature is introduced.

In one example, the operation **46** assumes the earlier-rolled out features are now familiar if a preselected number of days since the previous feature was introduced has passed.

In another example, the operation **46** actively measures familiarity by analyzing interaction of the user with the presented UI **28,** for example based on a usage of the medical device **10** by the specific user (e.g., in number of clicks or total time). When high, the next feature can be introduced. This time can differ amongst users, e.g., a user who hardly uses the system may have a slower rollout of features. The analysis of the user interaction in the operation **46** may also track a frequency of the use of a help function. When high, adding a new feature should be postponed.

Another factor analyzed by metric **46** may be a logic of the order the various already disclosed (i.e., rolled out) features are used. When they are used in a logical order, the user is probably more familiar with the system than when they are used in a random/illogical order and thus the user would be ready for a new feature to be added.

Another factor optionally analyzed by the metric **46** may be determining familiarity across multiple systems used by the clinician. For example, if the feature relates to displaying a new patient respiratory metric, if this has already been rolled out in one model of mechanical ventilator used by a clinician then this may increase the assessment of familiarity for that clinician. In a variant approach, the same feature might be rolled out on two or more different models (e.g., two different mechanical ventilator models) at the same time to provide the clinician with a smooth cross-model UI experience. This concurrent rollout can also reduce the frustration that could arise if the clinician likes and benefits from a feature rolled out on one device model but then finds it has not yet been rolled out on another device model.

If the medical device **10** includes a gaze tracker, then an eye direction/gaze can be analyzed. If the eyes seem to scan the whole screen / look in many directions the user can probably not find what he/she is looking for and thus needs to get more familiar with the already disclosed features before a new feature should be added. Note that the gaze tracking could be used even if the user input to the UI is not actually by gaze tracking (i.e., the gaze tracking is not an input to the user interface). For example, if a user frequently looks at a particular graph as indicated by gaze tracking, then that graph can thus be determined to be useful to the user, even though the user's frequent viewing of the graph does not constitute an input to the UI itself.

Another consideration that may be considered in the metric **46** may be whether the user is a fast learner or a slow learner; although learning speed can be automatically detected using approaches such as those just described, the met. c **46** could instead retrieve whether a user is a fast learner or a slow learner (or something in between) from user input (i.e., the user can select whether he is a fast or slow user). For fast learners, additions of features can go quicker and/or in larger chunks than for slow users.

Another factor the metric **46** could consider is whether an improvement in outcome for which the previous feature was meant has actually been achieved. For example, if the previously added feature was meant to reduce time to intervention and sometime after adding this feature indeed the time to intervention reduces (structurally/significantly), then this may be an indication that the feature is understood and used.

Another factor the metric **46** could consider is a calibration of feature use across the hospital staff. Studies suggest the existence of an Adoption Curve wherein about half of all users can be classified as Innovators (-2.5%), Early Adopters (-13.5%) or Early Majority (-34%). These are groups that are natural in adapting to change. The remainder of users can be classified as Late Majority (34%) and Laggards (16%) - these typically are users that are triggered by peer pressure, emerging norms or necessity. If such categorization information is available, it can be used in the operation **46** to tailor the rate of rollout of features to users based on their classification.

The metric **46** could optionally also employ a test (preferably on the same user interface **28,** preferably when the user is not busy with other things) about the previous feature(s), which, when passed (e.g., >80% correct answers) indicates that the user is ready for a next feature. Alternatively, a short questionnaire asking about the confidence in using/understanding the latest added feature(s).

The metric **46** might be (or employ) one or a combination of the above, (e.g., a new feature may only be implemented at least x days after introduction of the previous feature and no more scanning of the complete screen with the eyes.)

Furthermore, timing of adding a new feature can be influenced. There may be situations where adding a new feature at that point in time should be prevented. This is performed by a prevention module **48.** Some of the events monitored by the prevention module **48** can include alarms / criticality patient status; when an alarm is going off, it is not a good time to disclose a new feature, as the user should first handle the alarm. It is usually preferable to add new features in a non-critical situation (i.e., stable patient), so the user can explore it without being in a stressful situation. Another example is whether a user is busy with e.g., infusion pump or entering medication in EMR. The user should then not be distracted by a new feature being added. Another example is, depending on duration in the shift. In one embodiment, the new feature(s) (if any) would always only be added at the start of the shift to avoid surprises. In another embodiment, the new feature(s) (if any) would always be added only when already sometime in the shift has passed, to avoid disturbance during a period where the caregiver has already a lot of information to grasp (handover, understanding the patient situation).

As explained above, timing and/or size of the chunk to be added could be tailored to a specific user. For that, the medical device **10** needs to know which user is using the medical device **10.** This can be done for example by: using radiofrequency identification (RFID) login information; retrieving (e.g. from the EMR / work schedule) information on which user is responsible for the patient in the room of the medical device **10;** determining who is logged on to the medical device **10** or on other monitors in the same patient room; a face recognition with a camera; a proximity detection of a person (/person's mobile phone/badge) to the medical device **10,** e.g. by using Bluetooth technology to determine whose smart phones the user was close to with his/her own phone; a location tracking of hospital personnel using e.g. RFID tags; and so forth. Alternatively, the timing and selection of features to be added is not user-specific, but is the same on all medical monitors (of the particular type) throughout the hospital department.

The hospital department may have one or more "super users" (i.e., Innovators according to the Adoption Curve) of the medical device **10.** A "super user" is a person who knows the system well and to which others can turn for questions. This person might have been appointed by the hospital staff. Preferably it would be someone with technical skills, a fast learner, and often works with the system. Instead of getting appointed by other people, the system could also determine who would be the best candidate for super user. For example, selection can be based from one or more of the following: high usage of the system by the specific user (e.g., in number of clicks or total time), logic order of button presses, and/or eye direction/gaze often focused on the correct area on the screen. Preferably, those users will get a particular feature disclosed first before others get it disclosed. In that way, the super user can explore the feature and is ready for questions from others as soon as they get the feature disclosed.

As used herein, the term chunk is suitably a piece of software functionality that, without disruption of current functionality, can be added to the UI **28** in a single update. It is a part that can be added to the user interface in one go, without the need to add more to the UI **28** than that particular chunk; i.e., the medical device **10** would function well with the content that had been activated before, plus the chunk. A chunk contains one or more features. Each feature may consist of one or more functions / buttons / tabs / menus / tap & slide options / graphs, etc. Preferably, the chuck contains one or more logical combinations of functions / buttons / tabs / menus / tap & slide options / graphs / ... For example, if the chunk would contain a feature in which a caret for expanding and collapsing a graph is displayed, then it is logical that all graphs present would get a caret added (instead of only one graph of part of the graphs). The chunk would thus contain all these carets; those carets together can be seen as a cluster. A chunk can consist of one or more clusters.

The size/complexity of the chunk may also influence the time at which the next chunk will be added; if the chunk is very large or difficult, it will take longer to get used to the new feature(s), and thus adding a new chunk should wait a while.

As noted, there could be some default (pre-programmed) order for adding new features by the selection module **40.** Instead of a default order (possibly interrupted by features with urgency by the urgency module **42),** the medical device **10** might learn, while being in use, which is the best feature to add next.

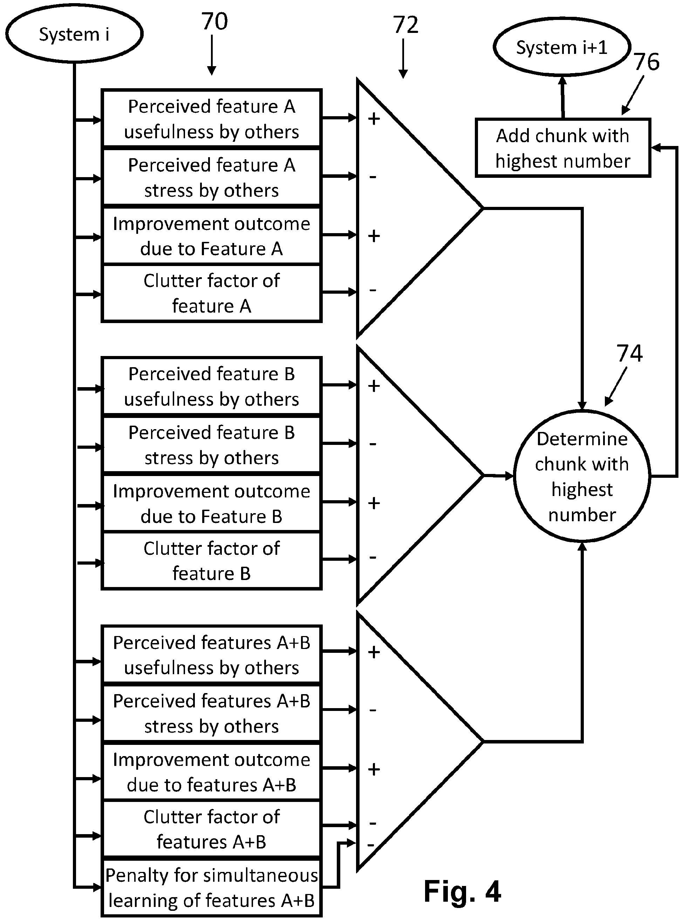

With reference now to Fig. 4, in one approach the selection of which chunk to activate next is done by evaluating how features were perceived by users that already had those features disclosed and/or how those improved the outcome for which the feature and/or complete medical device **10** was meant. Fig. 4 illustrates some suitable evaluation metrics **70** for an illustrative feature A, an illustrative feature B, and their combination A+B. To start with the latter: e.g. if the medical device **10** has as purpose to reduce the time to intervention, and after disclosing feature A with several users, their time to intervention reduced on average with 5 minutes, while for disclosing feature B it reduced on average with 15 minutes, then feature B seems to be the most useful feature and therefore feature B will, for other users, be disclosed first, before feature A will be disclosed to them. Besides using such outcome, as said, it could be used how features are perceived by other users. The start is the use of usability data to understand the speed of adaptation, the correct use and the use itself of the feature.

To further enrich the data, there can be a ranking process (e.g., with a questionnaire popping up) to determine how useful they find the feature, how much extra stress it gave right after disclosure, etc. Additionally or alternatively, automatic measures could be used for it, such as how often they used it and if they use it in a logical order with other button presses. Next to that, there might be a crowdedness measure, which relates to how many buttons, graphs, tabs, menus etc. are on the display, which has a negative influence. Also adding two features at once, instead of one, has a negative influence, because there is more to learn at once. All this information can be combined in logic **72** and used to estimate for the user under consideration, how his current medical device **10** plus feature A and/or plus feature B, and/or plus feature C, etc. would perform regarding improvement of outcome, stress to the user, perceived usefulness, etc. Based on that, the medical device **10** would in an operation **74** select which feature(s) to add, and the selected feature(s) are added in an operation **76** to come to the next iteration of the medical device **10** for that user. In case no improvement is expected, the medical device **10** is mature and will stay like this. Maturity also holds for the situation where there are no non-disclosed features left.

The new features may either be added without notifying the user, or could be added with a notification, such as a general message on the screen that a new feature has been added, optionally with a help button to go to the explanation of the new feature, or with arrows pointing to e.g. new buttons.

Instead of adding more and more features, feature removal is also possible by the illustrative removal module **50.** It could be either a manual action (where the user chooses which feature, he/she wants to remove) or the removal module **50** detects that a certain feature qualifies for removal. It could do this based on outcomes that did not improve after introducing the feature. Additionally or alternatively, it could do this based on the feature not being used for a long time (e.g., not being looked at, detected by eye tracking, or e.g., the particular button is never pressed). Since removal on such bases indicates that the feature is not useful, a removed feature might not get disclosed anymore for any other users (who did not have it disclosed yet). On the other hand, particular features are useful to have for some time and can be removed later, when its use has declined. Such features should also be disclosed to other users even if the first users do not use it anymore. An example is the "top contributors" that can be shown on an I pop-up. These top contributors are the parameters on which the I-number was based most at that moment. It is extra information for the user to get confidence in the I algorithm. The user might want to see this a couple of times, but after some time, the user would start to trust the I algorithm and does not need to see the underlying parameters anymore. Once the user has not been looking at the top contributors anymore already for a few times, this feature can be removed, resulting in the top contributors not showing up anymore. This prevents clutter on the UI **28** and the users can then focus more on other features that are more important.

To prevent culling of the provided features the urgency module **42** is used to add a weighing factor for the feature removal. When a feature is frequently added due to urgency by the urgency module **42,** the feature itself adds (clinical) value and should not be easily removed by a specific user. In addition, when the feature profile of a user has a large difference between the majority of the users in the same cohort of users (i.e. same hospital, same patient profile, same experience) the selection process by the selection module **40** and the feature removal module **50** is optimized to prevent a user bias.

There may be a digital tutorial (manual, (set of) instruction video(s), ...) of the medical device **10** that could for example be reached with a help button on the screen. The tutorial grows as the number of features grows, because each activated feature will turn up in the tutorial, while it would be hidden when it is not activated yet. The tutorial may nevertheless explain the still hidden features, but not in the main part of the tutorial; for example, for a manual, the hidden features are explained in an appendix. In that case, the feature moves from the appendix to the body of the manual as soon as it gets activated. When the user is interested in a feature that he/she reads in the appendix, and would like to have it already although it was not disclosed for him/her in the medical device **10** yet, then he/she could click on the item in the appendix to activate the feature in the system.

If software modules appear, outside the initial software, e.g. selected by a user from a "marketplace" containing software from different vendors, the medical device **10** would know which feature(s) of such a module the user could start with, because the medical device **10** knows which kind of features are already familiar to the user.

As used herein, the term "user" was used related to customizing the medical device **10** to the user. However, in many (hospital) settings, it might be preferable that the medical device **10** is the same for a user group. For example, the medical device **10** should be the same everywhere (i.e., on all patient monitors, if patient monitors are the illustrative medical device **10)** on the complete intensive care department of a hospital. The personnel, being the group of intensivists, residents, physician assistants, and nurses working on that ICU should then be seen as one user group. Instead of determining whether one single user is ready for a new feature, this will be determined for (the average of / median of / slowest learner of) the whole group. Ranking of which feature to add might be based on experience from other user groups, such as similar intensive care departments of other hospitals.

In a particular nonlimiting illustrative example of the operation **104** of grouping features of the update into chunks, groups of individual UI features, or features are related to different behavior of the same UI elements, can be grouped together. These features are clustered according to some pre-defined initial criteria, and based on this clustering/grouping the chunks are formed.

The grouping criteria can be driven by the nature/function of the features. For instance, trends analysis related features will typically belong to the same chunk. This grouping criteria can include a functionality-implementation dependent grouping. This is what is typically happening in software updates that are regularly happening in everyday solutions. This information is important, especially for interdependent features because they may not make sense, or may not work if they are introduced without one another. The dependency of the features to one another may be part of the feature description, which will be present in the data structure referred to.

In addition to implementation dependent clustering (i.e., chunk definition), other sets of parameters can also be considered when performing the clustering. These parameters are related to the medical device usage, medical device users, medical device context, or the workflow. The implementation and clinical context parameters can both be used in clustering the features, i.e., in defining the chunks (which can also be referred to as clusters, group of features, etc.).

For example, if there is a certain key performance indicator (i.e., timely septic shock detection) that is desired to be monitored or improved, the features corresponding to these can be selected, and chunks can be determined accordingly. In another example, if it is known that in three months an education of trend UI will be given, this workflow information (i.e., education planned) can be used to determine the chunks, and to activate the corresponding features, allowing the users to get familiar and play with the features before the education event. There is some dynamic context, patient, user, workflow information that is also being used in determining the chunks.

After the initial determination of the chunks, the first schedule is made with regard to how these will be activated (how they will transition from hidden to active). The performance criteria for the first to be introduced chunk is determined, including the type of data that will be collected, duration of data collection, and the analysis that will be applied, outputs that will be generated. In addition, for each chunk (not only the first one, but also for the others), criticality metrics related to each chunk needs are also determined. These can trigger re-grouping of features (i.e., re-creation of chunks) and/or updating of the chunk introduction order/rules/structure.

A first planned feature cluster (i.e., chunk A) is made active. This corresponds to a first pass of the operation **106** of Fig. 2. The performance criteria related to the chunk A is made active. The criticality metrics related to all chunks are made active. The criticality does not need to be urgent or short term (driven by patient fluctuations). It can also be a longer-term criticality (driven by performance measures). In a well-planned system (i.e., in common practice), longer-term planning will apply (i.e., for stability in UI and functionality, and do not want new UI elements being activated or deactivated based on daily patient fluctuations).

The active performance criteria related to chunk A is monitored during the UI presentation (operation **108** of Fig. 2) and evaluated. If it is determined that performance criteria of chunk A are satisfied (e.g., users are now familiar, the outcome improves, healthcare is more efficient, etc.) then a suitable update to the data structure is made in the operation **110,** and the process is repeated. Namely, (i) it is checked if the features need to be regrouped, in other words if the chunks need to be changed. (ii) the activation order, schema is updated, if necessary (iii), the performance criteria for a next chunk (i.e., chunk B) is updated/determined, (iv), the criticality criteria for all other chunks are updated/determined. Chunk B is activated, together with performance criteria, and criticality criteria.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A medical device **(10),** comprising:
a display **(24);**
an input interface **(12)** via which patient data is received; and
an electronic processor **(20)** implementing a user interface (UI) **(28)** in which a representation of the patient data is presented on the display, the electronic processor further programmed to perform an update of the medical device including:
receiving an update **(30)** for the medical device, the update comprising executable code for adding a plurality of features that modify the UI, the features being grouped into chunks;
generating a data structure **(32)** in which the plurality of chunks are assigned an active or hidden state;
progressively implementing the update of the medical device by:
presenting the UI modified by the features of the chunks assigned the active state and not modified by the features of the chunks assigned the hidden state; and
updating the data structure in response to an update trigger to reassign a triggered chunk of the plurality of chunks from the hidden state to the active state, whereby after the updating of the medical device the UI is presented modified by the features of the triggered chunk.

2. The medical device **(10)** of claim 1, wherein one of:
the medical device is a patient monitor, and the patient data comprise patient vital sign data received via the input interface **(12)** comprising a vital sign sensor interface,
the medical device is a mechanical ventilator, and the patient data comprise patient respiratory data received via the input interface comprising a respiratory sensor interface,
the medical device is a medical imaging device, and the patient data comprise patient medical images acquired by the input interface comprising imaging data acquisition hardware,
the medical device is a patient doctor management system, and the patient data comprise patient data retrieved from an electronic medical record or
the medical device is a medical records workstation, and the patient data comprise patient medical record data received via the input interface comprising a hospital information technology (IT) network.

3. The medical device **(10)** of either one of claims 1 and 2, wherein the progressive implementing the update of the medical device further includes:
updating the data structure **(32)** in response to an update trigger to reassign a triggered chunk of the plurality of chunks from the hidden state to the active state, whereby after the updating the UI **(28)** is presented including the features of the triggered chunk.

4. The medical device **(10)** of claim 3, wherein the progressive implementing the update of the medical device further includes:
analyzing usage of the presented UI **(28)** by a user; and
wherein the update trigger is generated by the analyzing of the usage of the presented UI by the user.

5. The medical device **(10)** of claim 4, wherein the progressive implementing the update of the medical device further includes:
based on the analysis of the usage of the presented UI (28) by the user, determining a criticality of a chunk to the usage of the presented UI by the user;
wherein the update trigger comprises the determined criticality satisfying a criticality trigger criterion.

6. The medical device **(10)** of either one of claims 4 and 5, wherein the progressive implementing the update of the medical device further includes:
removing the features of a chunk by reassigning the chunk from the active state to the hidden state in response to the analysis indicating usage of the chunk underruns a predetermined usage threshold.

7. The medical device **(10)** of either one of claims 5 and 6, wherein determining a criticality of a chunk to the usage of the presented UI **(28)** by the user includes:
analyzing at least one of a type of clinical cases performed and clinical cases scheduled with a feature list usage rate.

8. The medical device **(10)** of any one of claims 5-7, wherein the analysis of the usage of the presented UI **(28)** by a user includes:
tracking a gaze of the user interacting with the UI.

9. The medical device **(10)** of any one of claims 1-8, wherein the analysis of the usage of the presented UI **(28)** by a user includes:
determining a rate of consumption of the usage of the presented UI by the user.

10. The medical device **(10)** of any one of claims 3-9, wherein generating the update trigger comprises a predetermined dependency of the triggered chunk on another chunk that is reassigned from the hidden state to the active state.

11. The medical device **(10)** of any one of claims 1-10, wherein the data structure **(32)** assigns the chunks to the hidden or active states on a per user or per user group basis, and the method further includes:
identifying a user or user group of the UI **(28);** and
presenting the UI including the chunks assigned the active state for the identified user or user group and not including the chunks assigned the hidden state for the identified user or user group.

12. The medical device **(10)** of any one of claims 1-11, wherein the features of the plurality of chunks include one or more features in which the UI **(28)** presents additional information, one or more features in which the UI changes a presentation format of information, one or more machine-learning processes, and/or one or more features in which the UI provides user input for modifying operation of the UI.

13. The medical device **(10)** of any one of claims 1-12, wherein the data structure **(32)** is configured to:
analyze a plurality of users based on different medical systems that each user interacts with; and
determine, based on the analysis, the features of the chunks assigned the active state, and the features of the chunks assigned the hidden state.

14. A non-transitory computer readable medium **(16)** storing instructions readable and executable by an electronic processor **(14)** to perform a user interface (UI) updating method **(100)** for a medical device **(10),** the UI updating method comprising:
receiving an update **(30)** for a UI **(28),** wherein features of the update are grouped into a plurality of chunks;
assigning chunks of the plurality of chunks an active or hidden state; and
presenting the UI including the features of the chunks assigned the active state and not including the features of the chunks assigned the hidden state.

15. The non-transitory computer readable medium **(16)** of claim 14, wherein the UI updating method **(100)** further includes:
updating assignments of the chunks in response to an update trigger to reassign a triggered chunk of the plurality of chunks from the hidden state to the active state, whereby after the updating the UI **(28)** is presented including the features of the triggered chunk.
